# EUROPÄISCHE PATENTSCHRIFT

(11) **EP 3 120 817 B1**
(45) Veröffentlichungstag und Bekanntmachung des Hinweises auf die Patenterteilung: **01.07.2020**
(21) Anmeldenummer: 15002171.5
(22) Anmeldetag: 22.07.2015
(51) Int. Cl.: A61F 15/00, A61F 17/00, A61M 1/00, A61F 13/00, A61F 13/06, A61F 13/08, A61F 13/10, A61F 13/02

(54) **WUNDVERSORGUNGSANORDNUNG**
WOUND TREATMENT ASSEMBLY
SYSTEME DE TRAITEMENT DES PLAIES

(43) Veröffentlichungstag der Anmeldung: 25.01.2017
(73) Patentinhaber: Lohmann & Rauscher GmbH, 2525 Schönau/Triesting (AT)
(72) Erfinder: Schneider, Johannes, 80801 München (DE)
(74) Vertreter: Seranski, Klaus

(56) Entgegenhaltungen:
- EP-A1- 2 636 417
- WO-A1-2012/083934
- DE-A1-102012 205 408
- GB-A- 2 265 314

## Beschreibung

Die Erfindung betrifft eine Wundversorgungskit nach dem Oberbegriff des Patentanspruchs 1.

Wundversorgungsanordnungen gemäß dem Oberbegriff des Patentanspruchs 1 werden insbesondere im Rahmen der sogenannten Vakuumtherapie eingesetzt. Es hat sich gezeigt, dass im Besonderen die Heilung chronischer Wunden durch Anlegen von Unterdruck an diese Wunden gefördert werden kann. Dabei hat es sich weiter als vorteilhaft erwiesen, wenn die Wunde mit einem offenporigen Schaum oder Gaze als Füllmaterial abgedeckt bzw. gefüllt wird, die Wunde zur Erzeugung eines die Wunde und gegebenenfalls das Füllmaterial enthaltenden abgeschlossenen Wundraums abgedeckt und auf der der Wunde bzw. dem Füllmaterial abgewandten Seite der Abdeckeinrichtung ein Sauganschluss angebracht wird, über den der Wundraum mit einer zum Erzeugen von Unterdruck ausgelegten Saugeinrichtung verbunden werden kann. Bei anderen Anordnungen wird ein Flansch des Sauganschlusses von der Abdeckeinrichtung abgedeckt oder in einer eine Öffnung der Abdeckeinrichtung umlaufenden Tasche aufgenommen. Der Sauganschluss kann beispielsweise mit einem einerseits an einer beispielsweise in Form eines Rohrstutzens ausgeführten Verbindungseinrichtung des Sauganschlusses und andererseits mit einem an eine Saugeinrichtung anschließbaren Schlauch ausgestattet sein. Die Abdeckeinrichtung kann beispielsweise als folienartiges Material ausgeführt sein, welches an die der Wunde benachbarten Hautfläche luftdicht angelegt wird.

Wundversorgungsanordnungen, die im Rahmen der Vakuumtherapie einsetzbar sind, sind beispielsweise in der EP 0 620 720 B1 beschrieben.

In der DE 10 2009 019 646 A1 ist ein zur Verbesserung des Exsudat-Management zwischen dem Füllmaterial und dem Wundgrund einzulegende Kontaktschicht beschrieben, die einen Drainageraum zwischen dem Füllmaterial und dem Wundgrund bildet.

Im Rahmen der Vakuumtherapie einsetzbare Sauganschlüsse, welche über einen Schlauch mit einer Saugeinrichtung verbunden werden können, sind beispielsweise in der WO 03/073970 A1, der WO 2008/014358 A2 und der WO 2009/124548 A1 beschrieben. Ein als Saugkopf bezeichneter Sauganschluss mit zur Strömungsführung im Bereich der der Wunde zugewandten Begrenzungsfläche des Sauganschlusses dienenden Vorsprüngen ist in der EP 1 018 967 B1 beschrieben. Ferner ist ein Sauganschluss mit einer an das Füllmaterial anzulegenden Anlagefläche in Form einer scheibenartigen Schale in der EP 1 088 569 B1 angegeben. Bei einem in der WO 2010/008167 A2 beschriebenen Sauganschluss sind auf der dem Füllmaterial zugewandten Begrenzungsfläche durch Stege begrenzte Kanäle gebildet, durch die das Wundexsudat in Richtung auf eine Absaugöffnung geleitet werden soll.

In der WO 2010/011148 A1 ist eine im Rahmen der Vakuumtherapie einsetzbare Wundversorgungsanordnung beschrieben, die einen über eine Extremität des menschlichen Körpers ziehbaren undurchlässigen Schlauch sowie einen zwischen der Wunde und dem Schlauch anzuordnenden perforierten Körper aufweist. Mit dem perforierten Körper wird zwischen dem undurchlässigen Schlauch und dem Wundgrund ein Raum geschaffen, in dem über einen dichtend an den undurchlässigen Schlauch anlegbaren Schlauchanschluss ein Unterdruck erzeugt werden kann.

In der EP 1 162 932 B1 ist eine Wundversorgungsanordnung mit einer Umhüllung aus einem Kunststoffmaterial und einem in der Umhüllung enthaltenen fluidabsorbierenden Material beschrieben. Die in dieser Schrift beschriebene Wundversorgungsanordnung ist zum Schutz von Wunden gedacht. Sie ist mangels Schlauchanschluss für den Einsatz in der Vakuumtherapie nicht geeignet.

In der EP 2 636 417 A1 ist eine Wundversorgungsanordnung nach dem Oberbegriff des Patentanspruchs 1 angegeben. Die Wundversorgungsanordnung kann einen wasserdampfdurchlässigen Folienschlauch aufweisen, der zur Behandlung von Wunden an den Extremitäten, z.B. Fuß, Knöchel, Unterarm, Arm, Hand, über die Extremität gezogen und bezüglich der Wunde so positioniert wird, dass die Wunde mit der Abdeckeinrichtung dichtend abgedeckt wird. Im Anschluss daran kann die Abdeckeinrichtung mit der Haftfolie an der der Wunde benachbarten Haut befestigt werden. Dazu kann es vorgesehen sein, dass die Haftfolie von einem Wickel abgezogen und derart um ein Ende des Schlauchs gewickelt wird, dass sie einerseits an der Abdeckeinrichtung und andererseits an der Haut haftet. Der Offenbarungsgehalt der EP 2 636 417 A1 wird hinsichtlich der Eigenschaften von Haftfolien der Verfahren zum Anlegen von Wundversorgungsanordnungen somit durch ausdrückliche Inbezugnahme in diese Beschreibung aufgenommen. Diese Einzelheiten können in Kombination mit den Merkmalen des Hauptanspruchs zur Lösung der nachstehend angegebenen technischen Aufgabe beitragen. Es wird auch Schutz für entsprechende Merkmalskombinationen begehrt.

Das Einsatzgebiet bekannter Wundversorgungsanordnungen der eingangs beschriebenen Art ist begrenzt. Angesichts dieser Probleme im Stand der Technik liegt der Erfindung die Aufgabe zugrunde, das Einsatzgebiet von Wundversorgungsanordnungen, die für die Vakuumtherapie geeignet sind, zu erweitern.

Erfindungsgemäß wird diese Aufgabe durch ein Wundversorgungskit gemäß Patentanspruch 1 gelöst.

Folienschläuche derartiger Wundversorgungskits sind auch dann noch zur Wundversorgung einsetzbar, wenn die Wunde im Bereich eines sogenannten Fixateurs externe liegt. Dabei handelt es sich um ein durch die Haut von außen befestigtes Haltesystem, das dazu dient, einen Teil des Körpers ruhigzustellen. Die Erfindung beruht auf dem Gedanken, dass eine im Bereich eines Fixateur Externe anzulegende Wundversorgungsanordnung ohne Beeinträchtigung der Wundheilung über den gesamten Fixateur Externe gezogen werden kann. Dabei wurde im Rahmen der Erfindung erkannt, dass es für den Erfolg der Vakuumtherapie anders als in der EP 2 636 417 und der WO 2010/011148 A1 suggeriert nicht von entscheidender Bedeutung ist, die Abdeckeinrichtung an die Körperform anzupassen. Vielmehr kann ein Übermaß der Abdeckeinrichtung ohne weiteres in Kauf genommen werden, weil dieses Übermaß durch Anlegen des Unterdrucks an den Wunden so reduziert wird, dass es keine Beeinträchtigung der Wundheilung darstellt. Dabei kann die Abdeckeinrichtung ohne weiteres das außerhalb des Patienten freiliegende Haltesystem umschließen, so dass auch in diesem Bereich das Übermaß aufrechterhalten werden kann.

Als besonders zweckmäßig hat es sich erwiesen, wenn der Folienschlauch kreiszylindermantel- oder kegelstumpfmantelförmig ausgelegt ist, d.h. in einer Axialschnittebene rechteck- oder trapezförmig ausgeführt ist. Zusätzlich oder alternativ kann der Folienschlauch an einem axialen Ende an die Form eines Arms oder eines Beins angepasst sein. Er kann dann ohne weiteres mit der überdimensionierten Öffnung über beliebige Extremitäten gezogen werden, an die ein Fixateur Externe angelegt sein kann. Im Bereich der überdimensionierten Öffnung kann die Abdeckeinrichtung anschließend mit geeignetem Haftstreifen an der Haut dichtend befestigt werden. In diesem Zusammenhang wird Bezug genommen auf den Offenbarungsgehalt der EP 2 636 417 A1 (s.o.).

Dem Folienschlauch eines erfindungsgemäßen Wundversorgungskits kann eine Applikationshilfe wie etwa ein stabiler Ring mit einem an die Öffnungsumfangslänge angepassten Umfang oder ein Netz zugeordnet sein. Im Besonderen bei Einsatz einer Applikationshilfe in Form eines stabilen Rings kann die gewünschte Öffnungsumfangslänge auch durch Dehnen eines zur Herstellung des Folienschlauchs eingesetzten dehnbaren Materials, wie etwa eines Parafilms, erreicht werden.

Ein erfindungsgemäßes Wundversorgungskit ist besonders variabel zur Versorgung von Wunden im Bereich unterschiedlicher Extremitäten, die mit einem Fixateur Externe versorgt sind, anlegbar, wenn ein Folienschlauch ggf. geringer Dehnbarkeit über eine axiale Länge von 40 cm oder mehr, insbesondere 50 cm oder mehr, besonders bevorzugt 75 cm oder mehr eine Umfangslänge, d.h. eine Länge in einer senkrecht zur Achse verlaufenden Ebene, von 70 cm oder mehr, insbesondere 100 cm oder mehr aufweist.

Im Rahmen der Erfindung hat es sich weiter als zweckmäßig erwiesen, wenn der Folienschlauch eines erfindungsgemäßen Wundversorgungskits bei der Applikation in Abhängigkeit von den anatomischen Gegebenheiten konfektioniert wird. In diesem Zusammenhang ist es zweckmäßig, wenn der Folienschlauch mit einer sichtbaren Markierung, wie etwa einer das Anpassen an den Wundraum erleichternden und die axiale Länge entsprechender Folienschlauchabschnitte angebenden Längenmarkierungen ausgestattet ist.

Die bei der Behandlung mit Hilfe eines Fixateur Externe häufig beobachtete "Pin-Track-Infektion", d.h. eine Infektion der Weichteile um die Pinstellen, d.h. die Stellen, an denen das Haltesytem die Haut durchdringt, kann vermieden werden, wenn der Abdeckrichtung eine Fülleinrichtung zum Füllen eines bei Erzeugen eines Unterdrucks zwischen der Abdeckeinrichtung und der Haut des Patienten zwischen der Haut des Patienten, der Abdeckeinrichtung und einer die Haut des Patienten durchdringenden Stabilisierungseinrichtung entstehenden Hohlraums zugeordnet ist. So kann in dem gesamten Wundraum, einschließlich der Pinstelle, ein die Wundheilung fördernder Unterdruck aufrechterhalten werden.

Die Fülleinrichtung kann die Haut des Patienten durchdringende Stangen des Fixateur Externe umlaufen, wobei eine äußere Begrenzungsfläche der Fülleinrichtung kegelstumpfförmig ausgeführt sein kann. So kann die Fülleinrichtung einen das Einführen der Stangen des Fixateur Externe in einen die Fülleinrichtung axial durchsetzenden Hohlraum erleichternden Schlitz in einer Axialebene der Fülleinrichtung aufweisen.

Alternativ oder zusätzlich zu Fülleinrichtungen mit kegelstumpfmantelförmiger Begrenzungsfläche können Fülleinrichtungen in Form von verformbarer Gaze zum Einsatz kommen.

Zweckmäßigerweise ist der Folienschlauch eines erfindungsgemäßen Wundversorgungskits gegebenenfalls zusammen mit der Fülleinrichtung in einer sterilen bzw. sterilisierbaren Packung aufgenommen. Der Wundversorgungsschlauch kann dann in sterilem Zustand gelagert und eingesetzt werden. In diesem Zusammenhang hat es sich als besonders zweckmäßig erwiesen, wenn der Schlauch so in der Packung aufgenommen ist, dass die Entnahme des Schlauchs aus der Packung keine Probleme bereitet. Dazu kann der Folienschlauch in der sterilen Packung in geraffter Form oder auf einem Träger, wie etwa einem Kartonträger, in gewickelter Form aufgenommen sein. In der sterilen Packung können weitere Applikationshilfen wie Fäden, Ohren (Entnahme- bzw. Applikationshilfe) oder ähnliches enthalten sein. In diesem Zusammenhang ist beispielsweise daran gedacht, den Folienschlauch mit zusätzlichen "Ohren" zu versehen, an denen der Folienschlauch ohne Gefahr von Beschädigung angefasst und entnommen werden kann.

Zur Vermeidung von Beschädigungen des Folienschlauchs bei der Applikation und/oder während der Unterdrucktherapie ist es zweckmäßig, wenn der Folienschlauch gegebenenfalls durch separate Verstärkungsstreifen herstellbare Kontaktbereiche erhöhter Reißfestigkeit aufweist. Diese Kontaktbereiche können ohne Gefahr einer Beschädigung in Anlage an die außerhalb des Patienten freiliegenden Stabilisierungselemente des Fixateur Externe gelangen.

Ein erfindungsgemäßes Wundversorgungskit weist demnach eine Wundversorgungsanordnung sowie einen Fixateur Externe mit einem bei der Behandlung eines Patienten zumindest teilweise außerhalb des Patienten freiliegenden Stabilisierungselement auf, wobei der Folienschlauch der Wundversorgungsanordnung so dimensioniert ist, dass er den Fixateur Externe bei der Behandlung des Patienten vollständig aufnehmen kann.

Wenn beispielsweise ein Fixateur Externe zum Stabilisieren eines Unterarmbruchs eingesetzt wird und die Haut des Patienten durchdringende Stangen aufweist, die außerhalb des Patienten mit Hilfe einer Verbindungsstange gegebenenfalls variabler Länge verbunden werden, ist der Folienschlauch eines erfindungsgemäßen Wundversorgungskits so zu dimensionieren, dass er nicht nur den Unterarm des Patienten, sondern auch die den Unterarm durchdringenden Stangen und die Verbindungsstangen aufnehmen kann, so dass ein Unterdruck innerhalb des zwischen der Haut des Patienten und dem Folienschlauch gebildeten und den Fixateur Externe enthaltenden Raum erzeugt werden kann.

Ähnlich ist der Folienschlauch eines erfindungsgemäßen Wundversorgungskits zu dimensionieren, wenn der Fixateur Externe zum Fixieren eines Unter- oder Oberschenkelbruchs, eines Beckenbruchs, eines Handbruchs, eines Fußbruchs oder dergleichen eingesetzt wird. In jedem Fall ist dafür Sorge zu tragen, dass der Folienschlauch nicht nur das zu behandelnde Körperteil, sondern auch den entsprechenden Fixateur Externe bei der Behandlung aufnehmen kann.

Ein erfindungsgemäßes Wundversorgungskit kann Stabilisierungselemente in Form von Nägeln, Stiften oder dergleichen aufweisen. Zur Vermeidung einer Beschädigung des Folienschlauchs während Transport und Lagerung eines erfindungsgemäßen Wundversorgungskits können den Nägeln, Stiften, Schrauben oder dergleichen Abdeckungen oder Polsterungen zugeordnet sein. Dabei ist bei einer Ausführungsform der Erfindung daran gedacht, dass die Stabilisierungseinrichtung insgesamt durch eine Abdeckung oder Polsterung von dem Folienschlauch getrennt gehalten wird. Alternativ ist auch an solche Ausführungsformen gedacht, bei denen jedem einzelnen Stabilisierungselement, wie etwa jeder einzelnen Schraube, jedem einzelnen Nagel oder dergleichen eine einzelne Abdeckung bzw. Polsterung zugeordnet ist. Für den Fall, dass trotz der beschriebenen Maßnahmen Beschädigungen des Folienschlauchs auftreten, ist bei einer besonders bevorzugten Ausführungsform der Erfindung daran gedacht, dass das Wundversorgungskit neben den Stabilisierungselementen, dem Folienschlauch und gegebenenfalls den Fülleinrichtungen auch Reparaturmittel, wie etwa Spraypflaster, eine Gelmasse oder dergleichen zum Abdichten undichter Stellen in dem Folienschlauch aufweist. Die beschriebenen Fülleinrichtungen können auch in Form einer Gelmasse, wie etwa eines Wundsilikons, oder eines Füllkegels in dem Wundversorgungskit bereitgestellt werden.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
Fig. 1 ein Wundversorgungskit gemäß einer ersten Ausführungsform der Erfindung,
Fig. 2 ein Wundversorgungskit gemäß einer zweiten Ausführungsform der Erfindung,
Fig. 3 ein Wundversorgungskit gemäß einer dritten Ausführungsform der Erfindung,
Fig. 4 ein Wundversorgungskit gemäß einer vierten Ausführungsform der Erfindung,
Fig. 5 ein Wundversorgungskit gemäß einer fünften Ausführungsform der Erfindung, und
Fig. 6 ein Wundversorgungskit gemäß einer sechsten Ausführungsform der Erfindung.

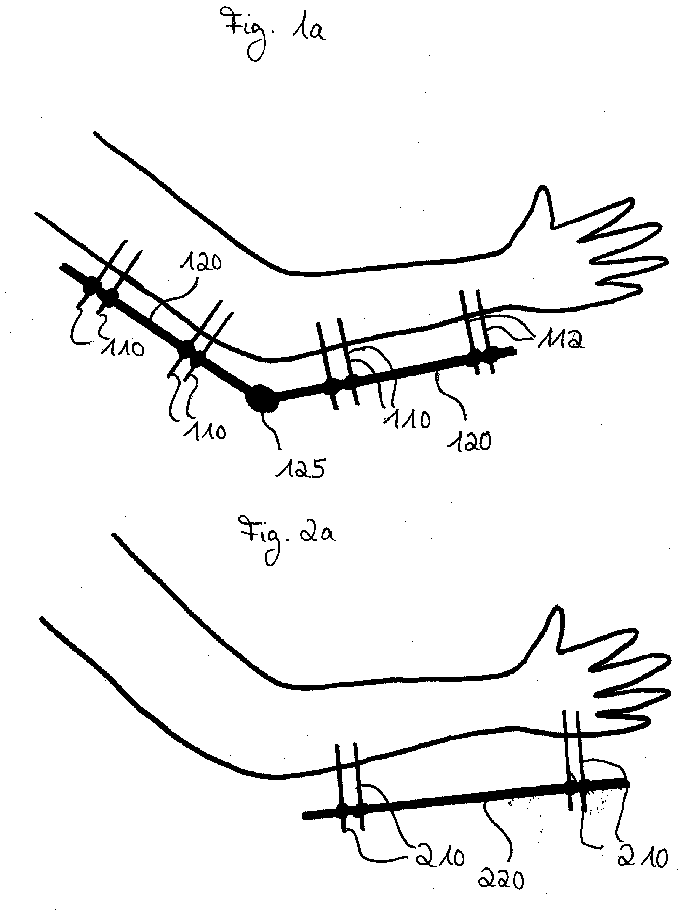

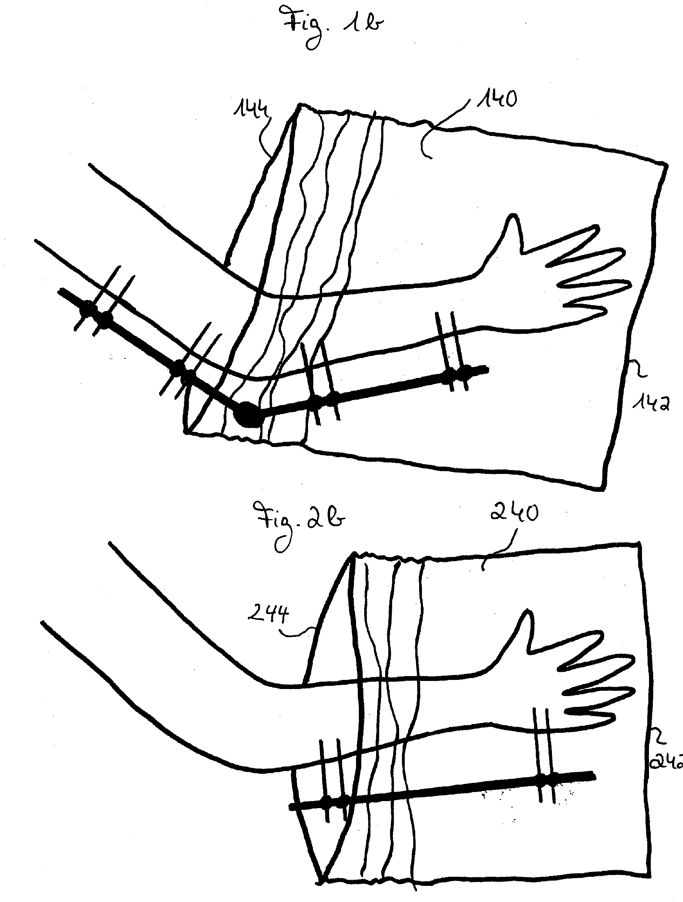

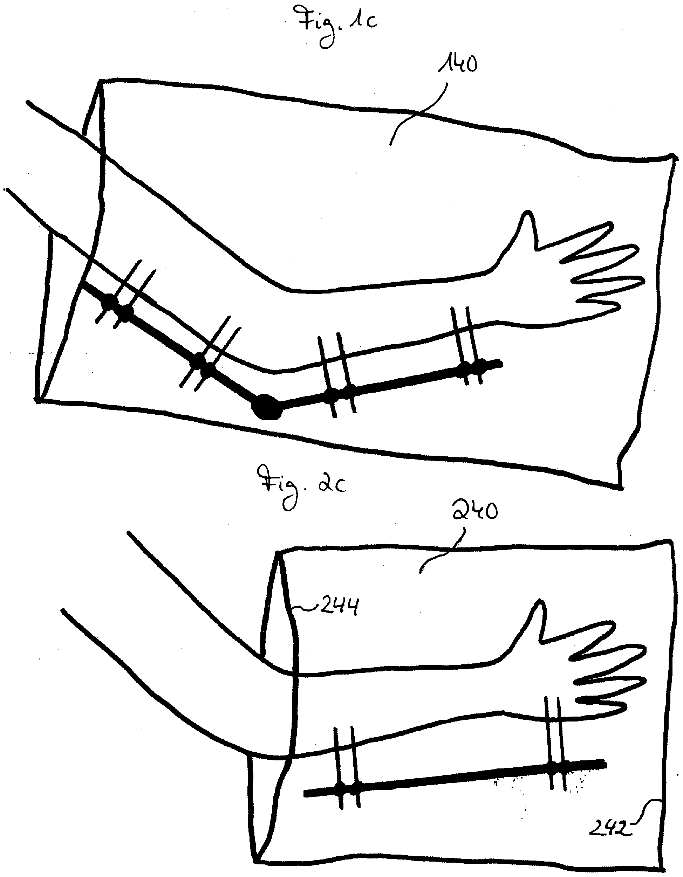

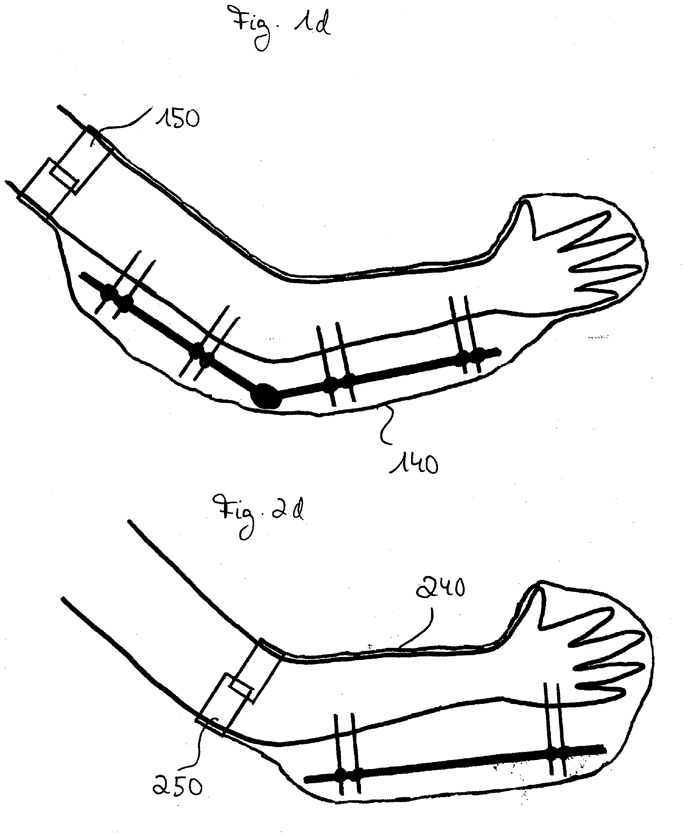

Das in Figur 1 dargestellte Wundversorgungskit umfasst insgesamt acht die Haut eines Patienten im Unterarm- und Oberarmbereich durchdringende Stabilisierungselemente in Form von Nägeln 112 sowie Verbindungsstangen 120, die über ein Gelenk 125 gelenkig miteinander verbunden sind. Die Verbindungsstangen 120 und das Gelenk 125 liegen außerhalb des Patienten frei und bilden einen Fixateur Externe, mit dem eine Fraktur im Unterarm-, Oberarm- und Ellenbogenbereich fixiert werden kann. Zur Behandlung einer im Bereich des Fixateur Externe liegenden Wunde wird ein an einem axialen Ende 142 luftdicht verschlossener Folienschlauch 140 aus einem wasserdampfdurchlässigen Material über den Arm des Patienten und den Fixateur Externe geschoben. Die dem luftdicht verschlossenen Ende 142 abgewandte Öffnung 144 des Folienschlauchs ist so dimensioniert, dass das Einführen der Extremitäten des Patienten in den Folienschlauch durch den Fixateur Externe nicht behindert wird. Der Vorgang des Einführens des Patientenarms in den Folienschlauch ist in den Figuren 1b und 1c dargestellt. Wenn die zu behandelnde Extremität einschließlich der zu behandelnden Wunde vollständig in dem Folienschlauch 140 aufgenommen ist, wird der die Öffnung 144 umlaufende Rand des Folienschlauchs mithilfe von Haftfolien 150 luftdicht an die Haut des Patienten angeschlossen, so dass zwischen der Haut des Patienten und dem Folienschlauch ein luftdicht, aber wasserdampfdurchlässig abgeschlossener Wundraum entsteht. In diesem Wundraum kann über einen Sauganschluss (nicht dargestellt) ein Unterdruck erzeugt werden, durch den die Wundheilung gefördert wird.

Die in Figur 2 dargestellte Ausführungsform der Erfindung unterscheidet sich lediglich dadurch von der anhand der Figur 1 dargestellten Ausführungsform, dass sie einen Fixateur Externe mit nur vier die Haut des Patienten durchdringenden Nägeln 210 und einer Verbindungsstange 220 aufweist. Der Fixateur Externe ist zum Fixieren eines Bruchs im Bereich des Handgelenks ausgelegt. Der Folienschlauch 240 der anhand der Figur 2 erläuterten Ausführungsform ist kürzer ausgeführt als der Folienschlauch 140 der anhand der Figur 1 erläuterten Ausführungsform, da er nur bis in den Ellenbogenbereich reichen muss. Dort wird der Folienschlauch 240, der an einem axialen Ende 242 luftdicht verschlossen ist, mithilfe von Haftfolien 250 luftdicht an die Haut des Patienten angeschlossen. So wird ein Wundraum erzeugt, der luftdicht, aber wasserdampfdurchlässig abgeschlossen ist und über einen Sauganschluss (nicht dargestellt) evakuiert werden kann.

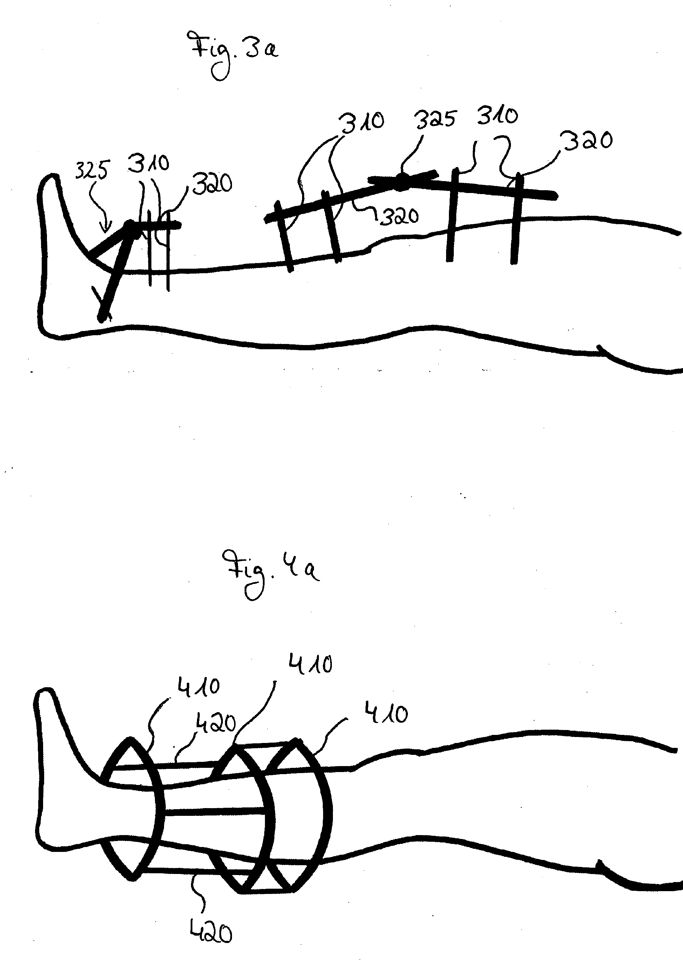

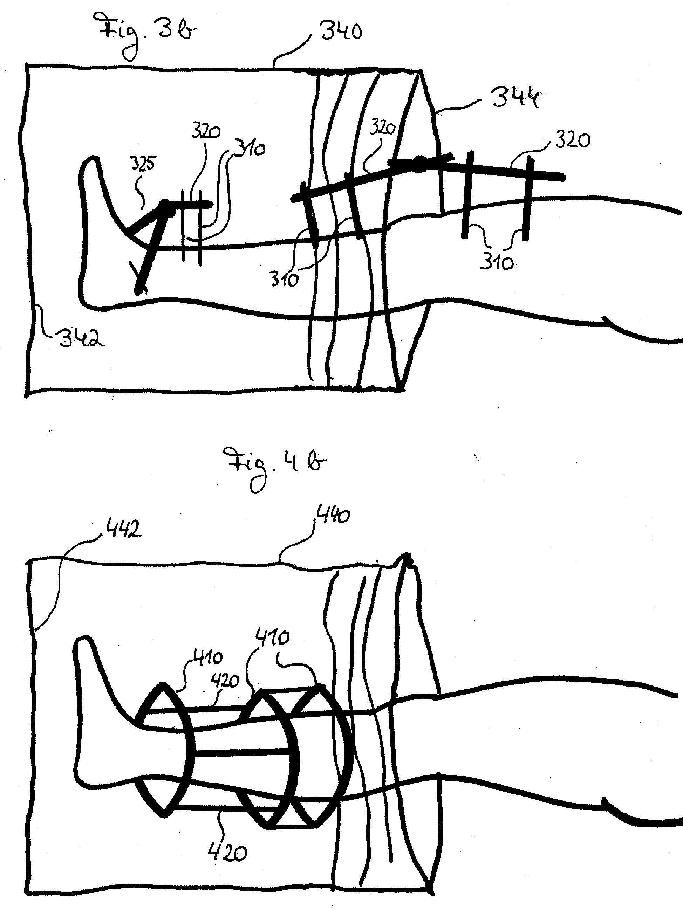

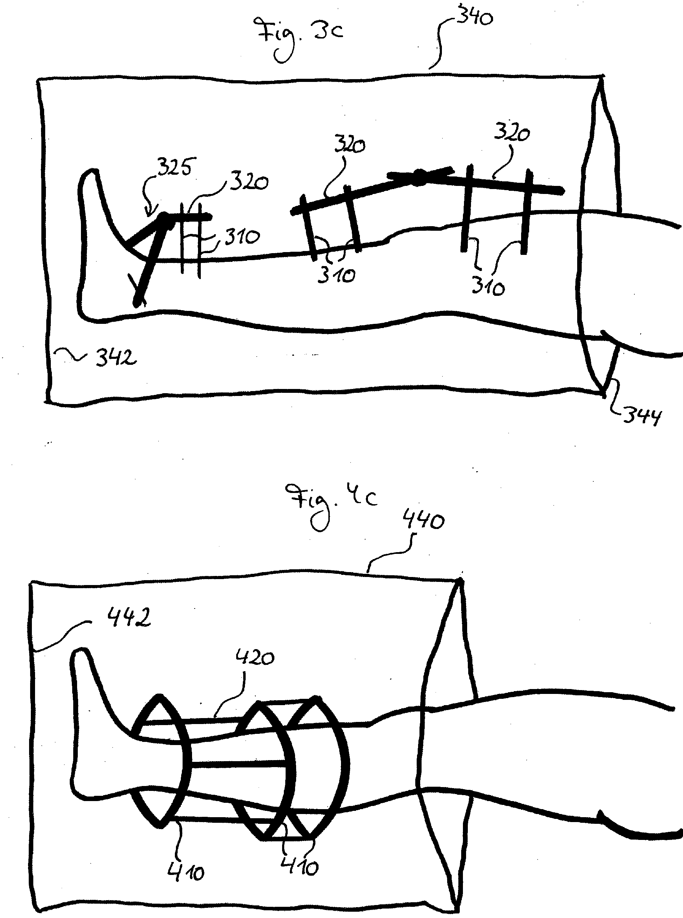

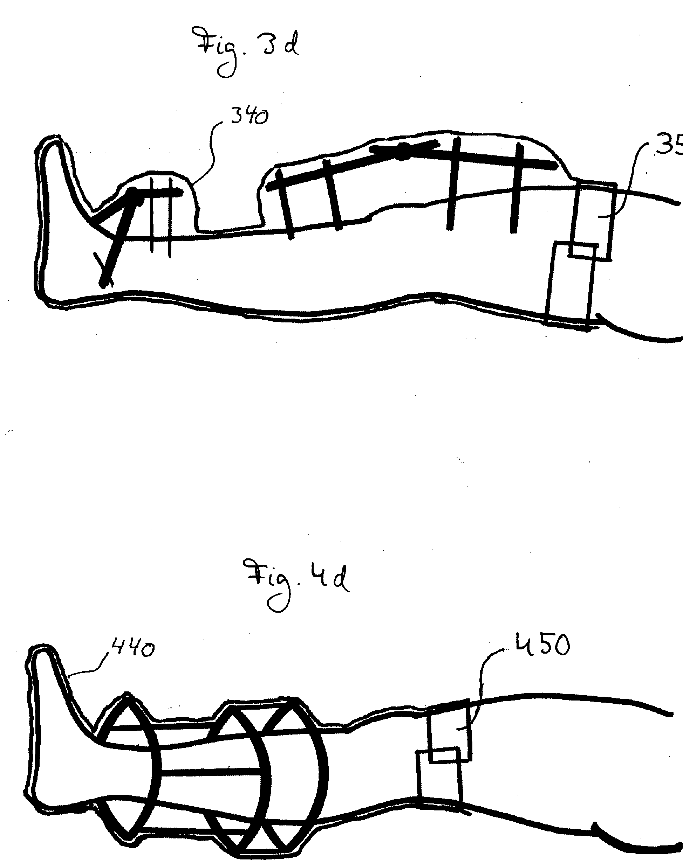

Die in Figur 3 dargestellte Ausführungsform unterscheidet sich im Wesentlichen dadurch von der anhand der Figuren 1 und 2 dargestellten Ausführungsform, dass sie zur Behandlung einer Wunde im Bereich eines Beins ausgelegt ist. Dabei umfasst die in Figur 3 dargestellte Ausführungsform der Erfindung insgesamt sechs die Haut des Patienten durchdringende Nägel 310 und drei Verbindungsstangen 320. Zwei der die Haut des Patienten durchdringenden Nägel sind im Bereich des Sprunggelenks des Patienten angebracht, wobei eine weitere Stabilisierungshilfe 325 das Sprunggelenk überbrückend vorgesehen ist. Die übrigen Nägel 310 sind beidseits des Kniegelenks des Patienten angebracht und über Verbindungsstangen 320 miteinander verbunden, die im Bereich des Kniegelenks gelenkig über ein Gelenk 325 miteinander verbunden sind. Ähnlich wie bei den anhand der Figuren 1 und 2 dargestellten Ausführungsformen der Erfindung umfasst das in Figur 3 dargestellte Wundversorgungskit ein an einem axialen Ende 342 luftdicht verschlossenen Folienschlauch 340, der so dimensioniert ist, dass er mit einer Öffnung 344 über das Bein und den Fixateur Externe gestülpt werden kann, wie in den Figuren 3b und 3c dargestellt. Ferner kann ein die Öffnung 344 umlaufender Rand mithilfe von Haftfolien 350 luftdicht an die Haut des Patienten angeschlossen werden, um so einen luftdichten, aber wasserdampfdurchlässigen Wundraum zu erzeugen, der über einen Sauganschluss (nicht dargestellt) evakuiert werden kann. Bei der anhand der Figur 3 dargestellten Ausführungsform der Erfindung beträgt die axiale Länge des Folienschlauchs mehr als 90 cm. Die Öffnungsumfangslänge im Bereich der Öffnung 344 des Folienschlauchs beträgt mehr als 120 cm.

Die in Figur 4 dargestellte Ausführungsform der Erfindung unterscheidet sich im Wesentlichen nur dadurch von der anhand der in Figur 3 dargestellten Ausführungsform, dass sie zum Behandeln einer Wunde im Bereich eines Unterschenkelbruchs vorgesehen ist. Der Fixateur Externe der in Figur 4 dargestellten Ausführungsform der Erfindung umfasst drei den Unterschenkel des Patienten umlaufende Stabilisierungsringe 410, die mithilfe von die Haut des Patienten durchdringenden Nägeln an den miteinander zu verbindenden Knochenfragmenten befestigt sind. Die umlaufenden Ringe 410 sind mithilfe von Verbindungsstangen 420 miteinander verbunden. Zum Erzeugen eines luftdicht abgeschlossenen Wundraums im Bereich des Unterschenkels wird ein an einem axialen Ende 442 luftdicht verschlossener Folienschlauch 400 über den Unterschenkel mit dem daran angebrachten Fixateur Externe gestülpt und mithilfe von Haftfolien 450 luftdicht an die Haut des Patienten angeschlossen. Dabei kann der luftdichte Anschluss an die Haut des Patienten gemäß Figur 4d oberhalb des Kniegelenks des Patienten erfolgen. Der Wundraum kann wie bei den übrigen Ausführungsformen der Erfindung mithilfe eines in der Zeichnung nicht dargestellten Sauganschlusses evakuiert werden, um so die Wundheilung zu fördern.

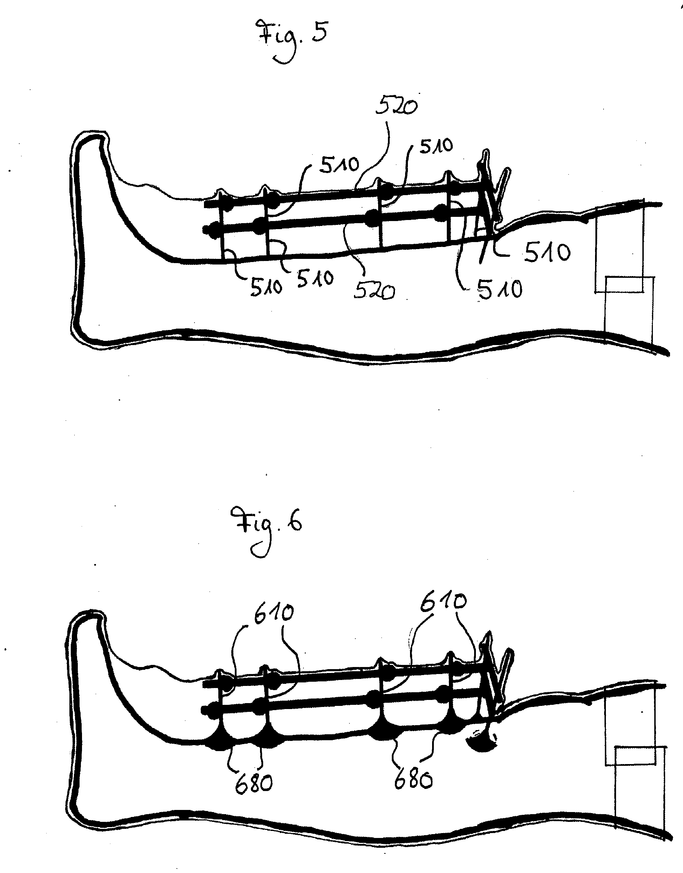

Die in Figur 5 dargestellte Ausführungsform der Erfindung unterscheidet sich im Wesentlichen nur dadurch von der anhand der Figur 4 dargestellten Ausführungsform, dass die die Haut des Patienten durchdringenden Nägel mithilfe von Verbindungsstangen 520 miteinander verbunden sind, ohne dass Stabilisierungsringe zum Einsatz kommen. Gemäß Figur 6 können im Bereich des Übergangs zwischen den Nägeln 610 und der Haut des Patienten kegelstumpfmantelförmige Fülleinrichtungen 680 vorgesehen sein, mit denen die Bildung eines Hohlraums zwischen den Nägeln, dem Folienschlauch und der Haut des Patienten verhindert werden kann.

Die Erfindung ist nicht auf die anhand der Zeichnung erläuterten Ausführungsbeispiele beschränkt. Vielmehr kann die Erfindung bei entsprechender Dimensionierung des Folienschlauchs auch zur Unterdruckbehandlung im Beckenbereich, Oberarmbereich, Oberschenkelbereich usw. eingesetzt werden.

## Patentansprüche

1. Wundversorgungskit mit
einer Wundversorgungsanordnung mit einer an der eine Wunde umgebenden Haut festlegbaren und zum Herstellen eines die Wunde enthaltenden abgeschlossenen Wundraums dienenden, zumindest abschnittweise wasserdampfdurchlässigen, einen Folienschlauch aufweisenden Abdeckeinrichtung und einem Sauganschluss, über den ein Unterdruck im Wundraum erzeugt werden kann, wobei der Folienschlauch eine Öffnung mit einer Öffnungsumfangslänge von 80 cm oder mehr, insbesondere 130 cm oder mehr aufweist, und
einem Fixateur Externe, der mindestens ein bei der Behandlung eines Patienten zumindest teilweise außerhalb des Patienten freiliegendes Stabilisierungselement aufweist, wobei der Folienschlauch so dimensioniert ist, dass er den Fixateur Externe bei der Behandlung des Patienten vollständig aufnehmen kann.

2. Wundversorgungskit nach Anspruch 1, **gekennzeichnet durch** Reparaturmittel, wie etwa Spraypflaster, eine Gelmasse oder dergleichen, zum Abdichten undichter Stellen in dem Folienschlauch.

3. Wundversorgungsanordnung nach Anspruch 1 oder 2, **gekennzeichnet durch** eine Fülleinrichtung, wie etwa eine Gelmasse, insbesondere Wundsilikon, und/oder mindestens einen ein Stabilisierungselement umlaufenden Kegel.

4. Wundversorgungskit nach einem der vorhergehenden Ansprüche, **dadurch gekennzeichnet, dass** der Folienschlauch kreiszylindermantel- oder kegelstumpfmantelförmig ausgeführt ist.

5. Wundversorgungsanordnung nach einem der Ansprüche 1 bis 3, **dadurch gekennzeichnet, dass** der Folienschlauch an die Form eines Arms oder Beins angepasst ist.

6. Wundversorgungsanordnung nach einem der vorhergehenden Ansprüche, **dadurch gekennzeichnet, dass** dem Folienschlauch eine Applikationshilfe, wie etwa ein an die Öffnungsumfangslänge angepasster Ring oder ein Netz zugeordnet ist.

7. Wundversorgungsanordnung nach einem der vorhergehenden Ansprüche, **dadurch gekennzeichnet, dass** der Folienschlauch über eine axiale Länge von 40 cm oder mehr, insbesondere 50 cm oder mehr, besonders bevorzugt 75 cm oder mehr eine Umfangslänge von 70 cm oder mehr, insbesondere 100 cm oder mehr aufweist.

8. Wundversorgungsanordnung nach einem der vorhergehenden Ansprüche, **dadurch gekennzeichnet, dass** der Schlauch mit einer sichtbaren Markierung, wie etwa einer das Anpassen an den Wundraum erleichternden und die axiale Länge angebenden Längenmarkierung, ausgestattet ist.

9. Wundversorgungsanordnung nach einem der vorhergehenden Ansprüche, **dadurch gekennzeichnet, dass** der Abdeckeinrichtung eine Fülleinrichtung zum Füllen eines bei Erzeugen eines Unterdrucks im Wundraum zwischen der Haut des Patienten, der Abdeckeinrichtung und einer die Haut des Patienten durchdringenden Stabilisierungseinrichtung entstehenden Hohlraums zugeordnet ist.

10. Wundversorgungsanordnung nach einem der vorhergehenden Ansprüche, **dadurch gekennzeichnet, dass** der Folienschlauch gegebenenfalls zusammen mit der Fülleinrichtung in einer sterilen Packung aufgenommen ist.

11. Wundversorgungsanordnung nach Anspruch 8, **dadurch gekennzeichnet, dass** der Folienschlauch in der sterilen Packung in geraffter Form oder auf einen Träger, wie etwa einen Kartonträger, gewickelter Form aufgenommen ist.

12. Wundversorgungsanordnung nach einem der vorhergehenden Ansprüche, **dadurch gekennzeichnet, dass** der Folienschlauch gegebenenfalls durch separate Verstärkungsstreifen herstellbare Kontaktbereiche erhöhter Reißfestigkeit aufweist.

## Claims

1. A wound care system comprising
a wound care arrangement with a means of covering which can be fixed to the skin surrounding the wound and creates an enclosed wound space containing the wound, with said covering having a tubular film and portions at least of the covering being water vapour-permeable, and with a suction connection by means of which a vacuum can be created in the wound space, wherein the tubular film has an opening with an opening circumference length of 80 cm or more, in particular 130 cm or more, and
an external fixator having at least one stabilising element which is at least partially exposed outside of a patient during treatment of the patient, wherein the tubular film is dimensioned to fully enclose the external fixator during treatment of the patient.

2. The wound care system according to claim 1, **characterised by** a repair means, such as a spray plaster, gel compound or similar for sealing leaks in the tubular film.

3. The wound care system according to claim 1 or 2, **characterised by** a filling device, such as a gel compound, in particular wound silicon, and/or at least a cone surrounding a stabilising element.

4. The wound care system according to one of the preceding claims, **characterised in that** the tubular film is designed as a sleeve with a circular cylinder shape or truncated cone shape.

5. The wound care system according to one of claims 1 to 3, **characterised in that** the tubular film is adapted to the shape of an arm or leg.

6. The wound care system according to one of the preceding claims, **characterised in that** an application aid, such as a ring adapted to the opening circumference length, or a mesh, is associated with the tubular film.

7. The wound care system according to one of the preceding claims, **characterised in that** the tubular film has a circumference length of 70 cm or more, in particular 100 cm or more over an axial length of 40 cm or more, in particular 50 cm or more, particularly preferably 75 cm or more.

8. The wound care system according to one of the preceding claims, **characterised in that** the tubular film has a visible marking, such as a length marking indicating the axial length and facilitating adaptation to the wound space.

9. The wound care system according to one of the preceding claims, **characterised in that** a filling device for filling a cavity created in the wound space between the skin of the patient, the covering and a stabilising device penetrating the skin of the patient when a vacuum is produced is associated with the covering.

10. The wound care system according to one of the preceding claims, **characterised in that** the tubular film is sterilely packaged, optionally together with the filling device.

11. The wound care system according to claim 10, **characterised in that** the tubular film is sterilely packaged in a gathered-up state or wound onto a carrier, such as a cardboard carrier.

12. The wound care system according to one of the preceding claims, **characterised in that** the tubular film may have contact areas with increased tear resistance optionally created by separate stiffening strips.

## Revendications

1. Ensemble de traitement des plaies comprenant :
un dispositif de traitement des plaies comprenant un moyen de recouvrement qui présente un film tubulaire et qui est perméable à la vapeur d'eau, au moins par sections, et qui permet de créer un espace fermé englobant la plaie du fait qu'il peut être fixé sur la peau qui entoure ladite plaie, et comprenant un raccord d'aspiration grâce auquel une pression négative peut être produite dans l'espace englobant la plaie, ledit film tubulaire présentant une ouverture d'une circonférence de 80 cm ou plus, notamment de 130 cm ou plus,
et un fixateur externe présentant au moins un élément de stabilisation exposé au moins partiellement à l'extérieur du corps du patient pendant le traitement du patient, ledit film tubulaire étant de dimensions lui permettant d'envelopper totalement ledit fixateur externe lors du traitement du patient.

2. Ensemble de traitement des plaies selon la revendication 1, **caractérisé par** un moyen réparateur, tel qu'un pansement en spray, une pâte gélatineuse ou similaire, permettant de colmater les défauts de fermeture du film tubulaire.

3. Dispositif de traitement des plaies selon la revendication 1 ou 2, **caractérisé par** un dispositif de remplissage, tel qu'une pâte gélatineuse, notamment de la silicone pour plaies, et/ou au moins un cône entourant l'élément de stabilisation.

4. Ensemble de traitement des plaies selon l'une des revendications précédentes, **caractérisé en ce que** le film tubulaire est conçu sous la forme d'une enveloppe de forme cylindrique circulaire ou tronconique.

5. Dispositif de traitement des plaies selon l'une des revendications 1 à 3, **caractérisé en ce que** le film tubulaire est adapté à la forme d'un bras ou d'une jambe.

6. Dispositif de traitement des plaies selon l'une des revendications précédentes, **caractérisé en ce qu'**un dispositif d'aide à l'application est associé au film tubulaire, tel qu'un anneau adapté à la circonférence de l'ouverture du film tubulaire ou une résille.

7. Dispositif de traitement des plaies selon l'une des revendications précédentes, **caractérisé en ce que** le film tubulaire présente une circonférence de 70 cm ou plus, notamment de 100 cm ou plus sur une longueur axiale de 40 cm ou plus, notamment de 50 cm ou plus, de préférence de 75 cm ou plus.

8. Dispositif de traitement des plaies selon l'une des revendications précédentes, **caractérisé en ce que** le film tubulaire est doté d'un marquage visible, tel qu'un marquage longitudinal indicateur de la longueur axiale et permettant de faciliter son adaptation à l'espace englobant la plaie.

9. Dispositif de traitement des plaies selon l'une des revendications précédentes, **caractérisé en ce qu'**un dispositif de remplissage est associé au moyen de recouvrement afin de remplir une cavité formée entre la peau du patient, le moyen de recouvrement et un dispositif stabilisateur traversant la peau du patient, pendant que l'espace englobant la plaie est soumis à une pression négative.

10. Dispositif de traitement des plaies selon l'une des revendications précédentes, **caractérisé en ce que** le film tubulaire est contenu dans un emballage stérile, éventuellement avec le dispositif de remplissage.

11. Dispositif de traitement des plaies selon la revendication 8, **caractérisé en ce que** le film tubulaire est contenu dans l'emballage stérile sous forme plissée ou sous forme enroulée sur un support, tel qu'un support cartonné.

12. Dispositif de traitement des plaies selon l'une des revendications précédentes, **caractérisé en ce que** le film tubulaire présente des zones de contact dotées d'une résistant accrue à la déchirure, lesquelles peuvent éventuellement être produites au moyen de bandes de renfort séparées.
